# EUROPEAN PATENT APPLICATION

(11) **EP 2 829 532 A1**
(43) Date of publication of application: **28.01.2015**
(21) Application number: 14185013.1
(22) Date of filing: 10.03.2008
(51) Int. Cl.: C07C 67/08, C07C 67/10, C07C 69/54, C07C 69/157, C07C 51/08, C07C 51/56, C07C 53/08, C07C 57/04

(54) **Hydroxyphenyl acrylate monomers and polymers**

(30) Priority: 12.03.2007 US 906445 P
(62) Divisional of application: 08250808.6
(71) Applicant: Rohm and Haas Company, Philadelphia, Pennsylvania 19106-2399 (US)
(72) Inventor: Benderly, Abraham, Elkins Park, PA 19027 (US)
(74) Representative: Kent, Venetia Katherine

(57) **Abstract**

New syntheses of phenolic acrylate monomers are provided as well as polymers comprising such phenolic acrylate monomers. Preferred polymers of the invention are useful as a resin component of chemically-amplified positive-acting resists.

## Description

In one aspect, the present invention relates to new syntheses of phenolic acrylate monomers. In another aspect, the invention relates to polymers comprising such phenolic acrylate monomers.

Photoresists are photosensitive films used for transfer of images to a substrate. A coating layer of a photoresist is formed on a substrate and the photoresist layer is then exposed through a photomask to a source of activating radiation. The photomask has areas that are opaque to activating radiation and other areas that are transparent to activating radiation. Exposure to activating radiation provides a photoinduced chemical transformation of the photoresist coating to thereby transfer the pattern of the photomask to the photoresist-coated substrate. Following exposure, the photoresist is developed to provide a relief image that permits selective processing of a substrate.

Photoresists are of interest that can be photoimaged with short wavelength radiation, including exposure radiation of 270 nm or less, such as wavelengths of 248 nm (provided by KrF laser). Use of such short exposure wavelengths can enable formation of smaller features. Accordingly, a photoresist that yields well-resolved images upon 248 nm exposure could enable formation of extremely small (e.g. sub-0.25 µm) features that respond to constant industry demands for smaller dimension circuit patterns, e.g. to provide greater circuit density and enhanced device performance.

While a variety of factors are involved in generating a highly resolved photoresist relief image, the resin component of a resist can have significant impact. Numerous efforts have been made to produce new resist polymers and monomers. SPIE, vol. 1466 Advances in Resist Technology and Processing (1991).

We have now found new syntheses of phenolic monomers and polymers that comprise such monomers.

More particularly, in one aspect, syntheses are provided for phenolic acrylate compounds such as those of the following Formula I: 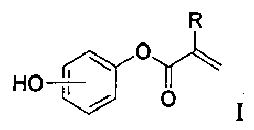 wherein R is hydrogen or C₁₋₆alkyl such as methyl or ethyl.

In another aspect, synthetic methods of the invention may suitably comprise reaction of a polyhydroxy phenyl compound such as a hydroquinone (C₆H₄-1-4-(OH)₂), catechol (C₆H₄-1-2-(OH)₂) or resorcinol (C₆H₄-1-4-(OH₂) with an acrylate anhydride such as methacrylic acid anhydride or acrylic acid anhydride. Preferably, the reaction is run in the presence of acid. In particular systems, an acrylate acid is employed such as methacrylic acid or acrylic acid. Suitably, the acid can serve as a solvent for the phenolic and anhydride reagents. Such systems can afford notable advantages including a reduced waste stream and the ability to recover unused reagents.

In a further aspect, an admixture of anhydride and acid for use in such synthetic methods is provided by reaction of an acid anhydride with an acrylic acid such as methacrylic acid or acrylic acid. In preferred systems, an acid anhydride such as acetic acid anhydride is reacted with a molar excess of an acrylic acid with removal such as by distillation of the lower molecular weight anhydride reaction product (i.e. acid).

In a yet further aspect, synthetic methods of the invention include use of a phenolic acrylate intermediate which undergoes an acid-ester exchange reaction to provide a phenolic acrylate compound. More particularly, a polyhydroxy phenyl compound such as a hydroquinone, catechol or resorcinol is reacted preferably in molar excess with an anhydride such as acetic anhydride to provide a phenyl compound comprising a hydroxyl group and an ester group (e.g. C₆H₄(OH)(ester)) including a monoacetate phenolic intermediate (e.g. (C₆H₄(OH)((OOCCH₃)) which then can be subjected to an acid-ester exchange by reaction with a suitable acid such as methacrylic acid or acrylic acid.

In a still further aspect, methods are provided for producing polymers using a monomer as described herein. Thus, a phenolic acrylate monomer is produced as disclosed herein and then reacted with one or more other monomers to provide the polymer.

In certain preferred aspects of the invention, methods to prepare polymers are provided where the polymer comprises repeat units that comprise a structure of the following Formula II: 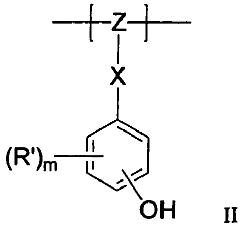 wherein in Formula II, Z is a bridge unit; X is one or more atoms; each R¹ is the same or different non-hydrogen substituent; and m is an integer of from zero (where no R¹ substituents are present) to 4.

Methods are further provided to prepare polymers that comprise repeat units that comprise a structure of the following Formula III: 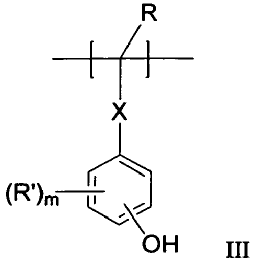 where in such Formula III R is hydrogen or alkyl such as C₁₋₆alkyl particularly methyl; and X, R¹ and m are the same as defined for Formula I above.

Preferred polymers may comprise additional repeat units distinct from spaced phenolic units such as those of Formulae I and II above, i.e. preferred polymers of the invention include copolymers, terpolymers, tetrapolymers, pentapolymers and other higher order polymers. Polymers of the invention can be particularly suitable for use as a resin component of a photoresist composition.

Other aspects of the invention are disclosed infra.

### Monomers and synthesis thereof

As discussed, preferred monomers produced by methods of the invention include those of the following Formula I: 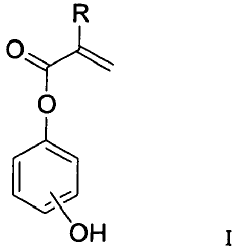 wherein R is hydrogen or optionally substituted alkyl such as optionally substituted C₁₋₆alkyl, and R is preferably hydrogen or methyl.

Particularly preferred monomers produced by methods of the invention include the following phenolic methacrylates (i), (ii) and (iii):

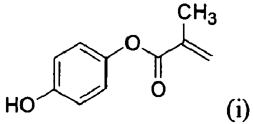

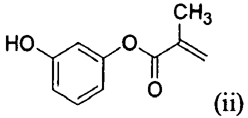

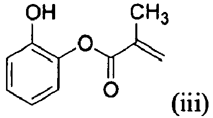

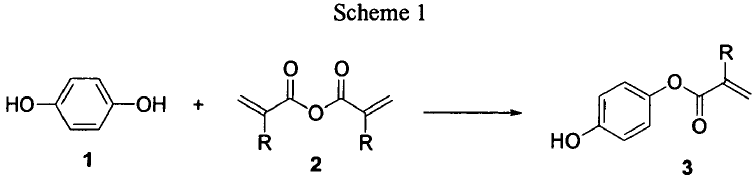

As discussed above and outlined in the exemplary Scheme 1, in one aspect, methods are provided which include reaction of a polyhydroxy phenyl compound such as hydroquinone **1** shown in Scheme 1 with a vinyl acid anhydride **2** in the presence of acid to provide a phenolic methacrylate compound **3.** In Scheme 1 above, the depicted "R" is the same as defined in Formula (I) above. A particularly preferred synthesis of Scheme 1 is exemplified in Example 1 below.

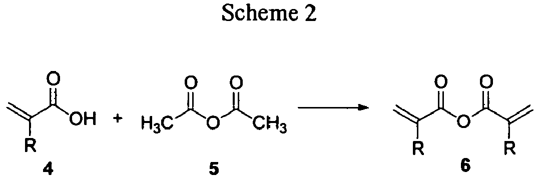

As outlined in the exemplary Scheme 2, in another aspect, methods are provided to produce a vinyl acid anhydride, which can be employed as reagent 2 in Scheme 1 above. More particularly, an acrylate acid 4 can be reacted with an acid anhydride 5, preferably in the presence of a further acid to provide a vinyl acid anhydride 6 to yield a phenolic methacrylate compound 3. In Scheme 2 above, the depicted "R" is the same as defined in Formula (I) above. A particularly preferred synthesis of Scheme 2 is exemplified in Example 2 below.

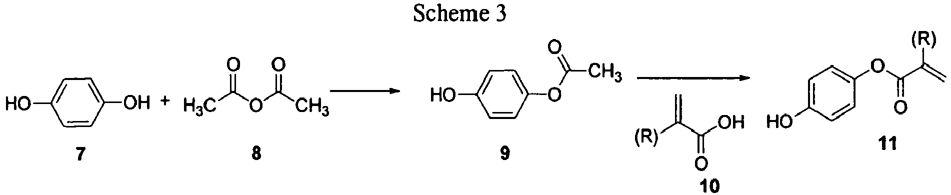

As discussed above and outlined in the exemplary Scheme 3, in a further aspect, methods are provided where a polyhydroxy compound **7** is reacted with an acid anhydride **8** preferably in the presence of acid to provide mono-ester phenolic intermediate **9.** If desired, excess starting materials may be recovered, and compound **9** is then reacted with acrylate acid **10** preferably in the presence of a further acid to provide phenolic methacrylate compound **11.** In Scheme 3 above, the depicted "R" is the same as defined in Formula (I) above. A particularly preferred synthesis of Scheme 3 is exemplified in Example 3 below.

The two-step process outlined in Scheme 3 can provide several notable advantages including use of comparatively inexpensive reagents (e.g., Ac₂O). Additionally, the process of Scheme 3 can facilitate synthesis of the acrylate compound **11,** i.e. where R of compound **11** is hydrogen

### Polymer syntheses

Polymers of the invention can be prepared by a variety of methods. In particular, polymers of the invention may be suitably formed by an addition reaction which may include free radical polymerization, e.g., by reaction of selected monomers (one of which includes a phenolic acrylate compound) to provide the various units as desired in the presence of a radical initiator under an inert atmosphere (e.g., N₂ or argon) and at elevated temperatures such as 70°C or greater, although reaction temperatures may vary depending on the reactivity of the particular reagents employed and the boiling point of the reaction solvent (if a solvent is employed). Suitable reaction solvents include e.g. tetrahydrofuran and ethyl lactate. Suitable reaction temperatures for any particular system can be readily determined empirically by those skilled in the art based on the present disclosure. A variety of free radical initiators may be employed. For example, azo compounds may be employed such as azo-bis-2,4-dimethylpentanenitrile. Peroxides, peresters, peracids and persulfates also could be employed. See the examples which follow for exemplary reagents and reaction conditions.

Suitable monomers for reaction to provide copolymers and other higher order polymers (e.g. terpolymers, tetrapolymers) of the invention include optionally substituted vinyl phenyl, optionally substituted styrene, optionally substituted alpha-methyl styrene, methacrylonitrile, acrylonitrile, 2-methyladamantylmethacrylate, 2-methyladamantylacrylate, or an alpha-butyrolactone methacrylate.

Other monomers that can be reacted to provide a polymer of the invention can be identified by those skilled in the art. For example, to provide acid labile groups, corresponding monomers can be reacted such as t-butymethacrylate, t-butylacrylate, t-butoxy methacrylate, t-butylmethacrylate; and ethoxyethyl methacrylate; to provide pendant acid groups corresponding monomers acid monomers can be reacted such as methacrylic acid and acrylic acid; and dissolution enhancers such as anhydrides which may be provided by reaction of suitable monomers such as itaconic anhydride and maleic anhydride.

Polymers of the invention suitably may have a wide ranges of molecular weights and molecular weight distributions. For instance, a polymer of the invention suitably may have a weight average molecular weight (M_{w}) of 1,000 to 100,000, more preferably 2,000 to 30,000, still more preferably from 2,000 to 15,000 or 20,000, with a molecular weight distribution (M_{w}/Mₙ) of 3 or less, more preferably a molecular weight distribution of 2 or less. Molecular weights (either M_{w} or Mₙ) of the polymers of the invention are suitably determined by gel permeation chromatography.

As discussed, various moieties described herein, including moieties of polymers and monomers may be optionally substituted. A "substituted" group may be substituted at one or more available positions, typically 1, 2, or 3 positions by one or more suitable groups such as e.g. halogen (particularly F, CI or Br); cyano; nitro; C₁₋₆alkyl sulfonyl such as mesyl; C₁₋₈ alkyl; C₁₋₈ alkoxy; C₂₋₈ alkenyl; C₂₋₈ alkynyl; hydroxyl; alkanoyl such as a C₁₋₆ alkanoyl e.g. acyl.

### Photoresist compositions

Polymers that comprises a polymerized monomer as disclosed herein can be particularly as a component of a photoresist composition, such as a chemically-amplified photoresist that is imaged with 248 nm exposure radiation. Such polymers may suitably comprise pendant alkyl ester or acetal polymer groups that can undergo photo-induced cleavage provide solubility differentials between exposed and unexposed areas of a resist coating layer.

Photoresists also generally contain a photoactive components such as one or more photoacid generators (i.e. "PAG") that are suitably employed in an amount sufficient to generate a latent image in a coating layer of the resist upon exposure to activating radiation. Suitable photoacid generators include imidosulfonates such as compounds of the following formula: 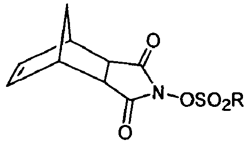 wherein R is camphor, adamantane, alkyl (e.g. C₁₋₁₂ alkyl) and perfluoroalkyl such as perfluoro(C_{1.2}alkyl), particularly perfluorooctanesulfonate and perfluorononanesulfonate. A specifically preferred PAG is N-[(perfluorooctanesulfonyl)oxy]-5-norbornene-2,3-dicarboximide.

Other optional photoresist additives include an added base such as an ammonium compound, which can enhance resolution of a developed resist relief image.

Use of photoresists in general is known such as described in U.S. Patent 7,105,266.

The following non-limiting examples are illustrative of the invention.

### Example 1: Preparation of methacrylic acid anhydride/methacrylic acid mixture

Acetic anhydride was added by dropwise addition to an excess of methacrylic acid (4:1) while simultaneously distilling acetic anhydride from the mixture. The reaction was carried out at 95Oc and 300 mmHg, catalyzed by 1 mole% amberlyst-15 (A-15), and inhibited with 3000 ppm PTZ and 1000 MEHQ pppm, 8% O₂.. At the end of the reaction, excess methacrylic acid was recovered by distillation under reduced pressure and the catalyst was recovered by filtration.

### Example 2: Monomer synthesis; preparation of hydroxyphenyl methacrylate

To a mechanically stirred solution of 2 moles of hydroxyquinone dissolved in 3 moles of methacrylic acid at 120°C and atmospheric pressure was added dropwise (30 min) 1 mole of methylacrylic acid anhydride and the mixture was maintaned at 120°C with stirring for additional 4 hrs (NMR analysis). Throughout the course of the reaction 8% oxygen was admitted to the system. At the end of the reaction, the methacrylic acid was recovered by distillation under reduced pressure (110°C and 200 mmHg), and the unreacted excess hydroquinone was precipitated out by the addition of toluene (1 liter) to the reaction mixture. A low level (1-2%) of the monomer 2-methyl-5-methylenehexanedioic acid was formede. This monomer was separated from the mixture by a washing step with distilled water. After phase separation, the desired 4-hydroxyphenyl methacrylate was obtained at 97% yield by the distillation of toluene under reduced pressure. {mp. (Uncorrected) 120°C; Anal. Calcd. For C₁₀H₁₀O₃: C, 67.41 : H, 5.66 ; O, 26.94. Found: C, 67.37 ; H, 5,62}.

### Example 3: Monomer synthesis; preparation of hydroxyphenyl methacrylate through mono-ester intermediate

A large excess of hydroquinone is reacted with acetic acid anhydride in the presence of acetic acid to provide the mono-acetate phenolic compound 1,4-C₆H₄(OH)(OOCCH₃)-Excess starting materials are recovered, and the intermediate compound 1,4-C₆H₄(OH)(OOCCH₃) is reacted with methacylic acid in the presence of acetic acid to provide hydroxyphenyl methacrylate.

### Example 4: Polymer synthesis

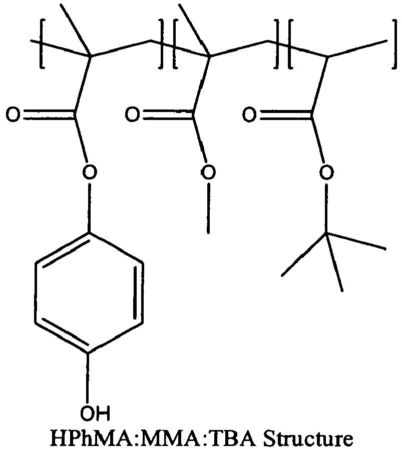

To a 500 mL, 3 neck round bottom flask equipped with a condenser, thermometer, magnetic stirrer and external oil heating bath, was added the following: 4-hydroxyphenyl methacrylate (HPhMA) (16.56 g, 0.093 mol), methyl methacrylate (MMA) (15.54 g, 0.155 mol), and tert-butyl acrylate (TBA) (7.95 g, 0.062 mol). Methanol (270 mL) was added and the resulting solution heated to reflux (67°C). Once at reflux, a solution of initiator 2,2'-azobis-2,4-dimethylpentanenitrile (1.54 g, 0.006 mol) in methanol (17.5 mL). The solution was held for 2 hours at reflux, after which another charge of initiator was added to the flask (0.77 g, 0.003 mol) in methanol (9 mL). The solution was held at reflux for 16 hours. After cooling, the polymer solution in methanol was washed with heptanes (3 x 300 mL). The solution was concentrated on a rotary evaporator to remove residual heptanes and then precipitated into DI water (2 liters). The wet cake was air dried for 24 hours and then dried at 60°C under vacuum for 18 hours. The yield was 90 %.

### Example 5-6: Additional polymer synthesis

Additional HPhMA:MMA:TBA terpolymers were prepared by the procedures of Example 3 above, but varying amount of monomers employed. In Table 1 below which follows Example 9, the ratio of each of the monomer units (as determined by ¹³C NMR analysis of the formed polymer), weight average molecular weight (Mw) and polydispersity (PD) are provided for the HPhMA:MMA:TBA terpolymers formed in Examples 5 and 6.

### Example 6: Additional polymer synthesis

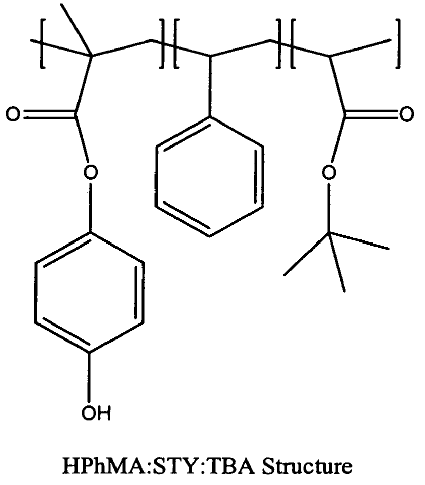

To a 500 mL, 3 neck round bottom flask equipped with a condenser, thermometer, magnetic stirrer and external oil heating bath, was added the following: 4-hydroxyphenyl methacrylate (HPhMA) (27.94 g, 0.157 mol), styrene (STY) (10.89 g, 0.105 mol), and tert-butyl acrylate (TBA) (11.17 g, 0.087 mol). Methanol (340 mL) was added and the resulting solution heated to reflux (67°C). Once at reflux, a solution of initiator 2,2'-azobis-2,4-dimethylpentanenitrile (1.73 g, 0.007 mol) in methanol (20 mL). The solution was held for 2 hours at reflux, after which another charge of initiator was added to the flask (0.87 g, 0.004 mol) in methanol (10 mL). The solution was held at reflux for 16 hours. After cooling, the polymer solution in methanol was washed with heptanes (3 x 300 mL). The solution was concentrated on a rotary evaporator to remove residual heptanes an then precipitated into DI water (2.5 liters). The wet cake was air dried for 24 hours and then dried at 60°C under vacuum for 18 hours. The yield was 90 %.

### Example 8-9: Additional polymer synthesis

Additional HPhMA:STY:TBA terpolymers were prepared by the procedures of Example 6 above, but varying amount of monomers employed. In Table 1 below, the ratio of each of the monomer units (as determined by ¹³C NMR analysis of the formed polymer), weight average molecular weight (Mw) and polydispersity (PD) are provided for the HPhMA:STY:TBA terpolymers formed in Examples 7 and 8.

**Table 1: Polymer Characterization**

| Example | Polymer Description | Composition (13C-NMR) | Mw | PD |
|---|---|---|---|---|
| 4 | HPhMA/MMA/TBA | 32/51/17 | 15400 | 2.7 |
| 5 | HPhMA/MMA/TBA | 42/40/18 | 18300 | 2.9 |
| 6 | HPhMA/MMA/TBA | 55/27/18 | 20200 | 3.1 |
| 7 | HPhMA/STY/TBA | 44/26/30 | 15300 | 2.6 |
| 8 | HPhMA/STY/TBA | 50/34/16 | 13100 | 2.3 |
| 9 | HPhMA/STY/TBA | 54/33/13 | 13700 | 2.3 |

### Example 10: Photoresist preparation and lithographic processing

A photoresist of the invention is prepared by admixing the following components in the specified amounts:

| Resist component | Amount |
|---|---|
| Resin | to provide 11.4 wt.% total solids liquid formulation |
| Photoacid generator | 3.5 wt. % of resin component |
| Basic additive | 0.1 wt. % of polymer |
| Surfactant | 0.05 wt. % of total solids. |

In that resist, the polymer is a HPhMA:MMA:TBA terpolymer prepared as described in Example 4 above. The photoacid generator of the resist is di-tertbutylphenyliodonium camphorsulfonate. The basic additive is the lactate salt of tetremethylammoniium hydroxide. The surfactant is the commercially available material sold under the name R08. The solvent is ethyl lactate.

That photoresist composition is spin coated onto 200 mm silicon wafers having a coating of an organic antireflective composition. The applied photoresist later is soft-baked at 90°C for 60 seconds and exposed through a photomask to 248 nm radiation. The exposed resist coating layer is then baked at 100°C for 90 seconds and developed using an alkaline aqueous developer.

## Claims

1. A method for producing a phenolic acrylate compound, comprising:
(a) reacting a polyhydroxy phenyl compound with acetic anhydride to provide an intermediate phenyl compound comprising ring substituents of hydroxyl and ester moieties;
(b) reacting the intermediate phenyl compound with an acrylate ester or acrylate acid compound to provide a phenolic acrylate compound.

2. The method of claim 1 wherein hydroquinone, catechol or resorcinol is reacted with acetic anhydride and the intermediate phenyl compound is reacted with methacrylic acid or acrylic acid.

3. The method of claim 1 wherein the intermediate phenyl compound is C₆H₄(OH)(ester).

4. The method of claim 1 further comprising reacting the phenolic acrylate compound with one or more other monomers to provide a polymer.
